# EUROPEAN PATENT SPECIFICATION

(11) **EP 1 684 851 B1**
(45) Date of publication and mention of the grant of the patent: **22.08.2007**
(21) Application number: 04811757.6
(22) Date of filing: 19.11.2004
(51) Int. Cl.: A61N 1/30

(54) **METHODS FOR PREPARING POLYMERIC BUFFER FORMULATIONS FOR ELECTROTRANSPORT APPLICATIONS**
VERFAHREN ZUR HERSTELLUNG VON POLYMEREN PUFFERFORMULIERUNGEN FÜR ELEKTROTRANSPORT-ANWENDUNGEN
PROCEDES DE PREPARATION DE FORMULATIONS DE TAMPON POLYMERE DESTINEES A DES APPLICATIONS D'ELECTROTRANSPORT

(30) Priority: 19.11.2003 US 523470 P
(43) Date of publication of application: 02.08.2006
(73) Proprietor: ALZA CORPORATION, Mountain View, CA 94039-7210 (US)
(72) Inventor: RAUSER, David, Gilroy, CA 95020 (US); PADMANABHAN, Rama, Los Altos, CA 94024 (US); PHIPPS, Joseph, B., Sunnyvale, CA 94087 (US); SUBRAMONY, Janardhanan, Santa Clara, CA 95051 (US)
(74) Representative: Williams, Paul Edwin
(86) International application number: PCT/US2004/039096
(87) International publication number: WO 2005/051484

(56) References cited:
- WO-A-96/34597
- WO-A-97/12644
- WO-A-02/089803
- US-A- 6 039 977
- US-A- 6 071 508

## Description

### FIELD OF THE INVENTION

The present invention relates to novel methods for preparing drug formulations for delivery by electrotransport that involve adjusting the pH of the drug formulation prior to incorporation into an electrotransport delivery system, and adding a buffering agent to the drug formulation upon incorporation into the electrotransport delivery system, wherein the buffering agent reduces changes in the pH of the drug formulation during electrotransport.

### BACKGROUND OF THE INVENTION

The delivery of active agents through the skin provides many advantages, including comfort, convenience, and non-invasiveness. In addition, gastrointestinal irritation and the variable rates of absorption and metabolism encountered in oral delivery are avoided. Transdermal delivery also provides a high degree of control over blood concentrations of any particular active agent.

Many active agents are not suitable for passive transdermal delivery because of their size, ionic charge characteristics, and hydrophilicity. One method for transdermal delivery of such active agents involves the use of electrical current to actively transport the active agent into the body through intact skin, which is known as electrotransport or iontophoretic drug delivery. In present electrotransport devices, at least two electrodes are used, which are disposed so as to be in intimate electrical contact with some portion of the skin. One electrode, called the active or donor electrode, is the electrode from which the active agent is delivered into the body. The other electrode, called the counter or return electrode, serves to close the electrical circuit through the body. In conjunction with the patient's skin, the circuit is completed by connection of the electrodes to a source of electrical energy, and usually to circuitry capable of controlling the current passing through the device. If the ionic substance to be driven into the body is positively charged, then the positive electrode (the anode) will be the active electrode and the negative electrode (the cathode) will serve as the counter electrode. If the ionic substance to be delivered is negatively charged, then the cathodic electrode will be the active electrode and the anodic electrode will be the counter electrode.

Electrotransport devices also require a reservoir or source of the active agent that is to be delivered or introduced into the body. Such reservoirs are connected to the anode or the cathode of the electrotransport device to provide a fixed or renewable source of one or more desired active agents. As electrical current flows through an electrotransport device, oxidation of a chemical species takes place at the anode while reduction of a chemical species takes place at the cathode. Both of these reactions generate a mobile ionic species with a charge state like that of the active agent in its ionic form. Such mobile ionic species are referred to as competitive species or competitive ions because the species compete with the active agent for delivery by electrotransport.

Many active agents exist in both free acid/base form and salt form. Although the salt forms of active agents are likely to have higher water solubility, the pH of an aqueous solution of the active agent salt may not be optimal from the standpoint of transdermal flux and stability of the drug at a particular pH. For example, human skin exhibits a degree of permselectivity to charged ions that is dependant upon the pH of the donor solution of an electrotransport device. For anodic donor reservoir solutions, transdermal electrotransport flux of a cationic species is optimized when the pH of the donor solution is about 4 to about 10, more preferably about 5 to about 8, and most preferably about 6 to about 7. For cathodic donor reservoir solutions, transdermal electrotransport flux of an anionic species is optimized when the pH of the donor solution is about 2 to about 6, and more preferably about 3 to about 5.

A problem that arises with the addition of pH-altering species (e.g., an acid or a base) to the active agent solution in an electrotransport device is that extraneous ions having the same charge as the active agent are introduced into the solution. These ions generally compete with the active agent ions for electrotransport through the body surface. For example, the addition of sodium hydroxide to raise the pH of a cationic active agent-containing solution will introduce sodium ions into the solution that will compete with the cationic active agent for delivery by electrotransport into the patient, thereby making the electrotransport delivery less efficient, i.e., less active agent will be delivered per unit of electrical current applied by the device.

To address this problem, methods have been developed for adjusting the pH of an active agent formulation prior to incorporation into an electrotransport delivery system that do not involve the introduction of extraneous ions. Such methods are described in U.S. Patent Nos. 6,071,508 and 5,853,383 and in PCT Application Publication No. WO 96/34597. The pH of active agent formulations often changes during electortransport, however, which adversely affects the stability of the active agent and reduces the efficiency of electortransport. The pH of active agents can also shift during long-term storage. A need exists in the art for methods and formulations that provide buffering capacity and reduce changes in the pH of active agents that occur during electrotransport and long-term storage.

### SUMMARY OF THE INVENTION

The invention relates to methods for preparing compositions for use in an electrotransport delivery system that comprise providing a drug solution comprising drug ions and associated counterions; adjusting the pH of the drug solution by contacting the drug solution with a first ion exchange material; separating the first ion exchange material from the pH-adjusted drug solution; adding the pH-adjusted drug solution to a reservoir of an electrotransport delivery system; and contacting the pH-adjusted drug solution with a second ion exchange material.

In certain embodiments of the invention, the drug ions are cationic, the associated counterions are anionic, the first ion exchange material is a polymeric anion exchange material, and the second ion exchange material is a polymeric anion or cation exchange material. In certain other embodiments of the invention, the drug ions are anionic, the associated counterions are cationic, the first ion exchange material is a polymeric cation exchange material, and the second ion exchange material is a polymeric cation or anion exchange material.

### BRIEF DESCRIPTION OF THE DRAWINGS

Figure 1 depicts the chemical structure of Compound 1, a 2-[3-[4-(4-piperidinyloxy)anilino]-1propenyl]benzamidine derivative.

Figure 2 depicts the iontophoretic flux of Compound 1 across heat-separated human epidermis (0.1 mA/cm²) from hydrogels loaded with a solution of Compound 1 pH-adjusted with either a polymeric resin or NaOH.

Figure 3 depicts buffering in hydrogels containing polacrilin and Compound 1.

### DETAILED DESCRIPTION OF ILLUSTRATIVE EMBODIMENTS

As used herein, the terms "adjusting," "adjust," and all variations thereof refer to changing by any measurable degree the pH of a solution or substance.

As used herein, the terms "contacting," "contact," and all variations thereof, refer to any means that directly or indirectly cause placement together of moieties, such that the moieties come into physical contact with each other. Contacting thus includes physical acts such as placing the moieties together in a container, combining the moieties, or mixing the moieties.

As used herein, the terms "drug" and "pharmaceutically active agent" refer to any chemical material or compound that induces a desired local or systemic effect, and can be delivered by electrotransport. The terms "drug ion" and "associated counterions" refer to either positively or negatively charged forms of a drug with which counterions of a charge opposite to that of the drug are associated.

Cationic drugs that can be used in the methods of the invention include any cationic drug that, when present in a formulation, has a first pKa, or any subsequent pKa, that is lower than the pH of the formulation buffering range. In addition, the cationic drug has at least one pKa that is lower than the desired storage or electrotransport operating pH and has at least one pKa that is higher than the storage or electrotransport operating pH. Examples of such cationic drugs include, but are not limited to, 2-[3-[4-(4-piperidinyloxy)anilino]-1propenyl]benzamidine derivatives such as, for example, ([3-(3-Carbamimidoyl-phenyl)-2-fluoro-allyl]-{4-[1-(1-imino-ethyl)-piperidin-4-yloxy]-phenyl}-sulfamoyl)-acetic acid hydrochloride; ([3-(3-Carbamimidoyl-phenyl)-2-methyl-allyl]-{4-[1-(1-imino-ethyl)-piperidin-4-yloxy]-phenyl}-sulfamoyl)-acetic acid hydrochloride; and ([3-(3-Carbamimidoyl-phenyl)-2-fluoro-allyl]-{3-carbamoyl-4-[1-(1-imino-ethyl)-piperidin-4-yloxy]-phenyl}-sulfamoyl)-acetic acid hydrochloride.

Anionic drugs that can be used in the methods of the invention include any anionic drug that, when present in a formulation, has a first pKa, or any subsequent pKa, that is higher than the pH of the formulation buffering range. In addition, the anionic drug has at least one pKa that is higher than the desired storage or electrotransport operating pH and has at least one pKa that is lower than the storage or electrotransport operating pH. Examples of such anionic drugs include, but are not limited to, captopril and lisinopril.

As used herein, the terms "separating," "separate," and all variations thereof refer to removing substantially all of the first ion exchange material from the drug solution.

As used herein, the terms "adding," and "add," and all variations thereof, refer to any means that directly or indirectly cause placement together of moieties or components, such that the moieties or components come into close proximity to each other. The terms include acts such as placing the moieties or components together in a container, combining the moieties or components, contacting the moieties or components, or stirring, vortexing, or agitating the moieties or components together.

As used herein, the terms "ion exchange resin" or "ion exchange material" refer to any material comprising (i) a mobile ionic species selected from the group consisting of hydronium and hydroxyl ions, and (ii) at least one oppositely charged, substantially immobile ionic species. The ion exchange materials useful in conjunction with certain embodiments of the invention are capable of donating either a hydroxyl ion (i.e., anion exchange materials or resins, which are typically used to adjust the pH of cationic drug formulations), or a hydrogen ion (i.e., cation exchange materials or resins, which are typically used to adjust the pH of anionic drug formulations).

As used herein, the terms "electrotransport" and "electrically-assisted transport" are used to refer to the delivery of drugs by means of an applied electromotive force to a drug-containing reservoir. The drug can be delivered by electromigration, electroporation, electroosmosis or any combination thereof. Electroosmosis has also been referred to as electrohydrokinesis, electroconvection, and electrically induced osmosis. In general, electroosmosis of a species into a tissue results from the migration of solvent in which the species is contained, as a result of the application of electromotive force to the therapeutic species reservoir, i.e., solvent flow induced by electromigration of other ionic species. During the electrotransport process, certain modifications or alterations of the skin can occur such as the formation of transiently existing pores in the skin, also referred to as "electroporation". Any electrically assisted transport of species enhanced by modifications or alterations to the body surface (e.g., formation of pores in the skin) are also included in the term "electrotransport" as used herein. Thus, as used herein, the terms "electrotransport" and "electrically-assisted transport" refer to (1) the delivery of charged drugs by electromigration, (2) the delivery of uncharged drugs by the process of electroosmosis, (3) the delivery of charged or uncharged drugs by electroporation, (4) the delivery of charged drugs by the combined processes of electromigration and electroosmosis, and/or (5) the delivery of a mixture of charged and uncharged drugs by the combined processes of electromigration and electroosmosis. The term "electrotransport delivery system" refers to any device that can be used to perform electrotransport.

As used herein, the term "competing ions" refers to ionic species having the same sign charge as the drug to be delivered by electrotransport, and which may take the place of the drug and be delivered through the body surface. Similarly, conventional buffering agents used to buffer the pH of a donor reservoir solution can likewise result in the addition of competing ions into the donor reservoir, which results in lower efficiency of electrotransport drug delivery.

The term "gel matrix," as used herein, refers to a composition, of which the reservoir of an electrotransport delivery device is generally comprised, having a viscosity of from about 1,000 to about 200,000 poise, preferably from about 5,000 to about 50,000 poise.

As used herein, the terms "reducing," "reduce," and all variations thereof, refer to decreasing by any measurable degree variations in the pH of a drug solution.

As used herein, the term "delivering" refers to the administration of a drug to a patient or test subject using electrotransport.

The term "patient," as used herein, refers to an animal, mammal, or human being.

The present invention relates to methods for preparing compositions for use in electrotransport delivery systems. The methods reduce changes in the pH of drug solutions during electrically assisted transport and during long-term storage without introducing competing ions that could negatively impact flux, resulting in effective delivery and stability of the drugs. The methods also prevent catalysis of drugs that have poor stability outside certain pH ranges by maintaining the pH of solutions of such drugs at a desired level. In certain embodiments, the methods of the invention involve a two-step process in which the pH of a drug solution is first adjusted using means that avoid the introduction of competing ions into the solution, and then a buffering agent is added to the pH-adjusted drug solution, which reduces changes in the pH of the drug solution during electrotransport or storage. In certain embodiments of the invention, the buffering agent used in the second step of the process is a polymeric resin.

In certain embodiments, the present invention is directed to methods that involve an initial adjustment of the pH of a drug solution prior to incorporating the solution into an electrotransport drug delivery system. The pH of any particular drug solution can be adjusted either upward or downward, as desired. In this way, the flux of the drug through the skin can be optimized, as can the stability of particular drug/polymer matrix compositions. In this regard, it has been found that partially or completely neutralized drug solutions can yield a higher transdermal flux than the corresponding drug salt formulation, particularly when the drug is a divalent or polyvalent species.

In contrast to prior methods used to adjust the pH of donor drug solutions prior to electrotransport delivery, the present technique does not involve the introduction of extraneous ions into the electrotransport system that would compete with the drug ions for electrotransport through the body surface. For example, with cationic drugs, partial or complete neutralization by admixture with potassium hydroxide, sodium hydroxide, or the like would result in the incorporation of potassium ions, sodium ions, or the like, into the drug formulation, species that would in turn compete with the cationic drug for electrotransport delivery and reduce the efficiency of drug delivery.

In certain embodiments, the present invention relates to methods in which the pH of a drug solution comprising drug ions and associated counterions is adjusted prior to incorporating the solution into an electrotransport drug delivery system. In certain embodiments of the invention, the pH of the drug solution is adjusted by contacting the drug solution with a first ion exhange material. In certain aspects, the invention is directed to methods in which the drug ions are cationic, the associated counterions are anionic, and the first ion exchange material is a polymeric anion exchange material. In preferred embodiments of the invention, the first polymeric anion exchange material is a polymeric anion exchange resin or a polymeric anion exchange membrane.

With cationic drugs, in certain embodiments of the invention, the first ion exchange material is preferably a polymeric anion exchange material that will exchange hydroxyl ions for the negatively charged counterions typically associated with cationic drugs, e.g., chloride, bromide, acetate, trifluoroacetate, bitartrate, propionate, citrate, oxalate, succinate, sulfate, nitrate, phosphate, and the like. Suitable anion exchange materials are typically the hydroxide forms of amine-containing polymers, e.g., polyvinyl amines, poly epichlorohydrin/tetraethylenetriamines, polymers containing pendant amine groups, and the like. A preferred anion exchange material for use herein is a co-polymer of styrene and divinyl benzene having a quaternary ammonium functionality and an associated hydroxyl ion. Other suitable anion exchange materials include, but are not limited to, the hydroxide forms of Amberlite^{™} IRA-958 (an acrylic/divinylbenzene copolymer available from Rohm and Haas), cholestyramine (a styrene/divinylbenzene copolymer also available from Rohm and Haas), Dowex 2X8 (a styrene/divinylbenzene available from Dow Chemical), and Macro-Prep High Q (an acrylic/ethyleneglycol dimethacrylate copolymer available from BioRad Laboratories). As will be appreciated by those skilled in the art, anion exchange materials containing primary, secondary and tertiary amines are relatively weak bases, while those containing quaternary amine functionalities are strongly basic, and will more quickly and effectively adjust upward the pH of formulations of cationic drug salts. Accordingly, such materials are preferred for use herein.

In certain aspects, the invention is directed to methods in which the drug ions are anionic, the associated counterions are cationic, and the first ion exchange material is a polymeric cation exchange material. In preferred embodiments of the invention, the first polymeric cation exchange material is a polymeric cation exchange resin or a polymeric cation exchange membrane.

With anionic drugs, in certain embodiments of the invention, the first ion exchange material used is preferably a cation exchange material that will exchange hydrogen or hydronium ions for the positively charged counterions typically associated with anionic drugs. Salts of cationic drugs are usually formed by treating the free acid form of the drug with a pharmaceutically acceptable base, typically an amine such as diethylamine, triethylamine, ethanolamine, or the like, giving rise to positively charged quaternary ammonium moieties associated with the drug. Cation exchange resins that will exchange hydrogen ions for such species include, for example, cation exchange resins comprising a polymer having one or more acid moieties. Such polymers include, for example, polyacrylic acids, polyacrylic sulfonic acids, polyacrylic phosphoric acids and polyacrylic glycolic acids. Cation exchange resins containing carboxylic acid moieties are weaker acids and are relatively more useful for buffering, while those containing functionalities such as sulfonic acids are more strongly acidic, and are accordingly preferred in connection with the present methods as providing faster and more efficient pH adjustment. Cation exchange resins of weaker acids useful for buffering include Amberlite IRP-64 (from Rohm and Haas) and acrylic polymers such as Bio-Rex 70 from Biorad.

When preparing drug solutions adapted for electrotransport delivery through human skin, the preferred direction and type of pH adjustment will depend upon whether the drug is cationic, and hence delivered from an anodic reservoir, or anionic and hence delivered from a cathodic reservoir, as well as on the solubility characteristics of the particular drug to be delivered. In general for electrotransport delivery through human skin, the pH of an anodic reservoir formulation is typically in the range of about 4 to about 10, more preferably in the range of about 5 to about 8, and most preferably from about 6 to about 7. In general for electrotransport delivery through human skin, the pH of a cathodic reservoir is typically in the range of about 2 to about 6, more preferably in the range of about 3 to about 5.

It will be appreciated by those skilled in the art that conventional ion exchange materials used as the first ion exchange material in the methods of the invention, e.g., cation and anion exchange resins, may be replaced with any relatively high molecular weight material having acid or base functionalities, such that conversion of ionized functionalities present in the drug molecule will be effected by exchange with protons or hydroxyl ions present in the material, and separation of the drug solution therefrom will be facilitated by virtue of the material's molecular weight. Generally, although not necessarily, it is preferred that the molecular weight of the material be at least about 200 Daltons, more preferably at least about 300 Daltons, and most preferably at least about 500 Daltons.

In certain embodiments, the present invention relates to methods in which the pH of a drug solution comprising drug ions and associated counterions is adjusted prior to incorporating the solution into an electrotransport drug delivery system. In certain embodiments of the invention, the pH of the drug solution is adjusted by contacting the drug solution with a first ion exhange material. In certain embodiments, the drug solution is contacted with the first ion exchange material by simple admixture of the first ion exchange material, typically in the form of an ion exchange resin associated with a solid support (e.g., beads or the like), with a solution of the drug salt. The relative quantities of the first ion exchange material and the drug salt will depend upon the desired change in pH, which is in turn dependent upon the degree of drug salt neutralization. Generally, the pH of the drug formulation will be adjusted such that the flux of the drug through the skin, during electrotransport drug delivery, is optimized. Accordingly, the preferred pH for any given drug salt formulation may be readily determined by conducting routine experimentation to evaluate optimum drug flux. For divalent or polyvalent drugs, neutralization is generally conducted to a degree effective to convert a substantial fraction of the drug salt, typically greater than about 80%, to a monovalent form.

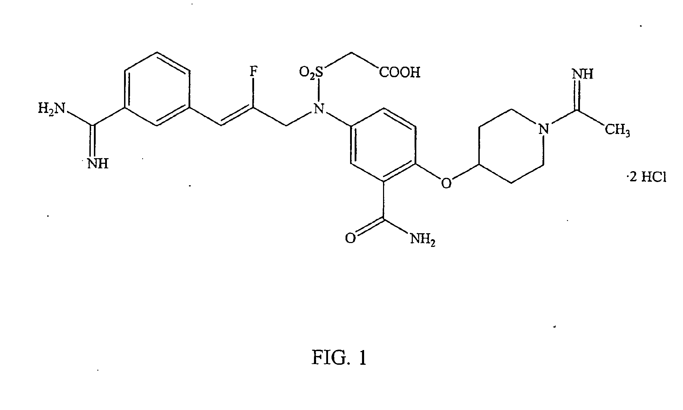

In certain embodiments of the invention, the drug is a cationic or anionic factor Xa inhibitor and an anti-coagulant. In preferred embodiments, the drug is a cationic benzamidine or naphthamidine derivative. In more preferred embodiments, the drug is a cationic benzamidine derivative. In still more preferred embodiments, the drug is a 2-[3-[4-(4-piperidinyloxy)anilino]-lpropenyl]benzamidine derivative as described, for example, in Japanese Patent Number JP 2003002832 and PCT Application Publication Number WO 02/089803, incorporated herein by reference in their entireties. In even more preferred embodiments, the drug is the 2-[3-[4-(4-piperidinyloxy)anilino]-1propenyl]benzamidine derivative depicted in Figure 1 and referred to as Compound 1. In certain embodiments of the invention, the drug is an anionic drug, such as, for example, captopril or lisinopril. In other embodiments of the invention, the drug is terbutaline.

Divalent and polyvalent drugs that can be used in certain embodiments of the methods and compositions of the invention include, but are not limited to, alniditan, as well as talipexole dihydrochloride, carpipramine dihydrochloride, histamine dihydrochloride, proflavine dihydrochloride and gusperimus trihydrochloride.

The concentration of the drug in the formulations prepared by the methods of certain embodiments of the invention depends upon the delivery requirements for the drug. The percent drug loading can range, for example, from about 1 % to about 30 %, more preferably from about 1.5 % to about 20 %, and more preferably from about 2 % to about 10 %.

Reaction between the drug solution and the first ion exchange material is typically quite fast, on the order of minutes. After the reaction is allowed to proceed to completion, the drug solution may be separated from the first ion exchange material using centrifugation, standard filtration techniques (e.g., filters, screens, etc.), or using a syringe and a narrow gauge (e.g., 26 gauge) needle.

The pH-adjusted drug solution can then be introduced into the reservoir of an electrotransport delivery system, typically by incorporation into a gel matrix material that serves as the drug reservoir. Certain embodiments of the invention relate to methods for preparing compositions for use in an electrotransport delivery system in which the pH-adjusted drug solution is contacted with a second ion exchange material. In some embodiments of the invention, the pH adjusted-drug solution is first added to the reservoir of an electrotransport delivery system, and the second ion exchange material is then added to the reservoir containing the drug solution. In other embodiments of the invention, the pH-adjusted drug solution is first contacted with the second ion exchange material, and the resultant mixture is then added to the reservoir of an electrotransport delivery system. In still other embodiments of the invention, the second ion exchange material is first added to the reservoir of an electrotransport delivery system, and then the pH-adjusted drug solution is added to the reservoir containing the second ion exchange material.

In certain embodiments of the invention, the drug ions are cationic, the associated counterions are anionic, and the second ion exchange material is a polymeric anion or cation exchange material. As understood by those of ordinary skill in the art, the choice of the appropriate second polymeric ion exchange resin is determined by the acid/base properties of the resin. For certain formulations prepared according to the methods of the invention, a polymeric anion exchange material provides the desired properties, and for certain other formulations prepared according to the methods of the invention, a polymeric cation exchange material provides the desired properties.

In certain embodiments of the invention, the drug ions are anionic, the associated counterions are cationic, and the second ion exchange material is a polymeric anion or cation exchange material. Again, as understood by those of ordinary skill in the art, the choice of the appropriate second polymeric ion exchange resin is determined by the acid/base properties of the resin.

In certain embodiments, the second polymeric anion or cation exchange material is a polymeric anion or cation exchange resin. In other embodiments, the second polymeric anion or cation exchange material is a polymeric anion or cation exchange membrane. Suitable second polymeric cation exchange resins comprise, for example, polacrilin, acrylate, methyl sulfonate, methacrylate, carboxylic acid functional groups, sulfonic acid, sulfoisobutyl, or sulfoxyethyl. In particularly preferred embodiments, the second polymeric cation exchange resin comprises polacrilin or acrylate.

Suitable second polymeric cation exchange resins comprise, for example, a polymer having one or more acid moieties. Such polymers include, for example, polyacrylic acids, polyacrylic sulfonic acids, polyacrylic phosphoric acids and polyacrylic glycolic acids. For buffering using anion exchangers, those ion exchange materials identified as weak anion are preferred. Several such polymers are available within the "Bio-Rex" and "AG" family of resins from Biorad (i.e. Bio-Rex 5 and AG 4-X4). Other anion exchange materials include forms of Amberlite (available from Rohm and Haas), e.g., Amberlite IRA67. Further anion exchange resins include the "Dowex" family of resins from Dow Chemicals (Dowex Monosphere 77).

The degree of neutralization of the second ion exchange resin in the formulations prepared according to certain embodiments of the methods of the invention, and the concentration of the second ion exchange resin, are spread over a range of values. In certain embodiments of the invention, the degree of neutralization of the second polymeric anion or cation exchange resin is about 2 % to about 70 %. In more preferred embodiments, the degree of neutralization of the second polymeric anion or cation exchange resin is about 5 % to about 50 %. In even more preferred embodiments, the degree of neutralization of the second polymeric anion or cation exchange resin is about 5 % to about 30 %.

The concentration of the second polymeric anion or cation exchange resin in the formulations prepared according to certain embodiments of the methods of the invention is about 20 meq/mL to about 200 meq/mL. In more preferred embodiments, the concentration of the second polymeric anion or cation exchange resin is about 20 meq/mL to about 140 meq/mL. In even more preferred embodiments, the concentration of the second polymeric anion or cation exchange resin is about 25 meq/mL to about 60 meq/mL. The lower end of the percentage of the second ion exchange resin is preferred because it adds the least amount of competing sodium ions to the formulation. Furthermore, at lower concentrations, adverse effects in achieving steady state flux are avoided with shorter rise time to attain steady state flux.

In certain preferred embodiments of the invention, the drug is a 2-[3-[4-(4-piperidinyloxy)anilino]-1propenyl]benzamidine derivative and the second ion exchange resin is polacrilin. The desired amount of the polacrilin in the formulations prepared according to such embodiments of the methods of the invention is about 0.23 % to about 1.13 %, neutralized to between about 5 % to about 10 %. For tighter pH control during delivery, in certain embodiments of the invention, this range would narrow down to about 0.23 % to about 0.40 % of the second anion exchange resin, neutralized to between about 5 % and 8 %.

It will be appreciated by those working in the field that formulations prepared by the present methods can be used in conjunction with a wide variety of electrotransport drug delivery systems, as the methods are not limited in any way in this regard. For examples of electrotransport drug delivery systems, reference may be had to U.S. Pat. Nos. 5,147,296 to Theeuwes et al., 5,080,646 to Theeuwes et al., 5,169,382 to Theeuwes et al., and 5,169,383 to Gyory et al, the disclosures of each of which are incorporated by reference herein in their entireties.

The reservoir of the electrotransport delivery devices generally comprises a gel matrix, with the drug solution uniformly dispersed in at least one of the reservoirs. Suitable polymers for the gel matrix can comprise essentially any nonionic synthetic and/or naturally occurring polymeric materials. A polar nature is preferred when the active agent is polar and/or capable of ionization, so as to enhance agent solubility. Optionally, the gel matrix can be water swellable. Examples of suitable synthetic polymers include, but are not limited to, poly(acrylamide), poly(2-hydroxyethyl acrylate), poly(2-hydroxypropyl acrylate), poly(N-vinyl-2-pyrrolidone), poly(n-methylol acrylamide), poly(diacetone acrylamide), poly(2-hydroxylethyl methacrylate), poly(vinyl alcohol) and poly(allyl alcohol). Hydroxyl functional condensation polymers (i.e., polyesters, polycarbonates, polyurethanes) are also examples of suitable polar synthetic polymers. Polar naturally occurring polymers (or derivatives thereof) suitable for use as the gel matrix are exemplified by cellulose ethers, methyl cellulose ethers, cellulose and hydroxylated cellulose, methyl cellulose and hydroxylated methyl cellulose, gums such as guar, locust, karaya, xanthan, gelatin, and derivatives thereof. Ionic polymers can also be used for the matrix provided that the available counterions are either drug ions or other ions that are oppositely charged relative to the active agent.

In certain embodiments of the invention, the reservoir of the electrotransport delivery system comprises a hydrogel. In other embodiments of the invention, the reservoir comprises a non-hydrogel, dry matrix.

In certain preferred embodiments of the invention, the reservoir of the electrotransport delivery system comprises a polyvinyl alcohol hydrogel as described, for example, in U.S. Patent No. 6,039,977, incorporated herein by reference in its entirety. Polyvinyl alcohol hydrogels can be prepared, for example, as described in U.S. Patent No. 6,039,977. The weight percentage of the polyvinyl alcohol used to prepare gel matrices for the reservoirs of the electrotransport delivery devices, in certain embodiments of the methods of the invention, is about 10 % to about 30 %, preferably about 15 % to about 25 %, and more preferably about 19 %.

Incorporation of the drug solution into the gel matrix can be done any number of ways, i.e., by imbibing the solution into the reservoir matrix, by admixing the drug solution with the matrix material prior to hydrogel formation, or the like.

Thus, after adjusting the pH of the drug solution using the methods of the invention and either before or after contacting the pH-adjusted solution with the second ion exchange material, the solution is incorporated into the drug reservoir, e.g., a gel matrix as just described, and is then administered to a patient using an electrophoretic drug delivery system. The second ion exchange material serves to reduce changes in the pH of the drug reservoir, and to maintain the desired pH of the reservoir, during electrotransport, resulting in greater stability and enhanced delivery of the drug.

In certain embodiments of the invention, a pH-adjusted drug solution is contacted with a second ion exchange material, and the solution is then stored, rather than being incorporated into the reservoir of an electrotransport delivery device for administration to a patient. The second ion exchange material serves to reduce changes in the pH of the drug solution and to maintain the desired pH of the solution during long-term storage. Drug solutions formulated according to the methods of the invention can thus be stably stored for extended periods of time such as, for example, weeks to months to years.

In certain other embodiments of the invention, a pH-adjusted drug solution is introduced into the reservoir of an electrotransport delivery system, and is contacted with a second ion exchange material, either before or after incorporation into the reservoir. Instead of being used immediately, the reservoir is stored for an extended period of time. The second ion exchange material serves to reduce changes in the pH of the reservoir and to maintain the desired pH of the reservoir during storage. Reservoirs containing a pH-adjusted drug solution prepared according to the methods of the invention can be stably stored for extended periods of time such as, for example, weeks to months to years.

The following examples are illustrative of certain embodiments of the invention and should not be considered to limit the scope of the invention.

### EXAMPLE 1: Preparation of Cationic Drug Formulations for Electrotransport

The pH of a concentrated solution of a 2-[3-[4-(4-piperidinyloxy)anilino]-1propenyl]benzamidine derivative depicted in Figure 1, and referred to as Compound 1, was adjusted by adding either NaOH or small quantities of a hydroxylated anion exchange resin (AG1-X8) to the drug solution. Exchange of the chloride counterion of the drug molecule with hydroxide from the resin raised the pH of the drug solution without introducing any competing ion that could reduce drug flux during electrotransport. After the pH of the drug solution was adjusted to the desired value, the resin was removed by filtration through a syringe filter (0.2 µm).

Hydrogels were typically prepared by placing polyvinyl alcohol (PVOH) at 19 wt % in purified water at 90°C for 30 minutes, reducing the temperature to 50°C, dispensing the gel suspension into disks, and freeze-curing. The formed hydrogels were then allowed to imbibe the pH-adjusted drug solution as a concentrated aqueous solution at room temperature to obtain the desired drug loading. Alternatively, drug loading was achieved by adding the pH-adjusted solution of the drug to the PVOH hydrogel solution before freezing. In the thermally processed formulations, PVOH was dissolved in purified water at 90°C. After reduction of the temperature to 50°C, an aqueous pH-adjusted solution of the drug was added to the PVOH solution and allowed to mix for 30 minutes. The PVOH-drug mixture was dispensed into disks and freeze-cured. Finished hydrogels were used in flux studies or extracted with purified water for drug-stability analysis.

To maintain the pH of the hydrogel during electrotransport, different buffers were incorporated into the hydrogel either directly or through the drug loading solution. Polacrilin (Amberlite^{®} IRP-64), an anion exchange resin with a carboxylic acid functional group, was added directly to the hydrogel solution during thermal processing at varied weight percents.

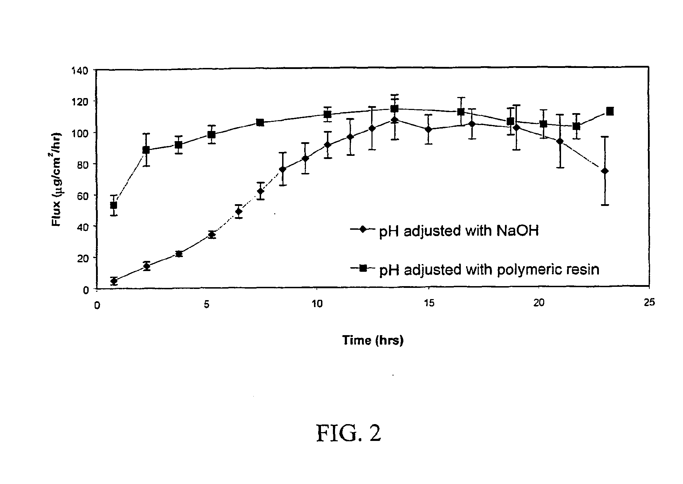

When NaOH was initially used to adjust the pH of the drug solution, steady state flux was typically achieved after a 10 - 12 hour delay. The use of an anion exchange resin to initially adjust the pH of the drug solution eliminated the introduction of competing sodium ions into the drug hydrogel. As a result, the time to reach steady state flux was reduced to about 3 - 4 hours as shown in Figure 2.

When a polymeric (polacrilin or acrylate) buffer was incorporated into the hydrogels, the pH was maintained to within 0.21 of the initial value for a 30% neutralized polacrilin buffer at a concentration of 95 meq/ml during *in vitro* electrotransport for 24-hour at a current density of 0.1 mA/cm². With all formulations, steady state flux was similar to that seen with unbuffered hydrogels. Buffering capacities of the polymeric buffers were a function of the degree of neutralization. Shifts in pH were reduced as the degree of neutralization increased. Various ionic strengths were used at differing degrees of neutralization in flux studies (in-vitro) without major consequence to drug flux. Table 1 shows the effect of the degree of neutralization with respect to polymer ionic strength. As the degree of neutralization increased, the pH shift was less dramatic in all cases.

**Table 1: pH shifts in hydrogels buffered with polacrilin containing Compound 1 at varied ionic strength and neutralization of the polymeric resin (n=3).**

| Buffer Concentration (meq/mL) | Percent Neutralization | pH Shift |
|---|---|---|
| 28 | 5 | 0.38 |
| 28 | 10 | 0.7 |
| 28 | 30 | -0.04 |
| 57 | 5 | -0.38 |
| 57 | 30 | 0.85 |
| 44 | 6 | -0.78 |
| 95 | 30 | 0.21 |
| 140 | 25 | 0.77 |

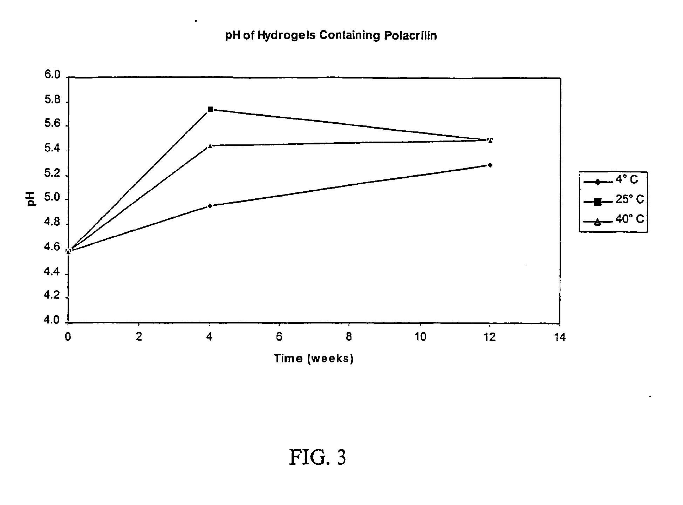

In addition, hydrogels prepared as described above and containing polacrilin and Compound 1 were stored at 4°C, 25°C, or 40°C for 12 weeks and the pH of the hydrogels were determined each week. Figure 3 shows the changes in the pH of the hydrogels that occurred over time, and demonstrates that the polacrilin served to buffer the hydrogels during storage.

### EXAMPLE 2: Preparation of Anionic Drug Formulations for Electrotransport

The pH of an anionic drug is adjusted with a polymeric cation exchange material and is then buffered with a polymeric ion exchange material according to the following procedure.

The drug ceftriaxone (supplied as the disodium salt) has the following three pKa's: 3 (carboxylic), 3.2 (amine), and 4.1 (enolic OH), which act as bases in solution. Using the Henderson-Hasselbach equation, the theoretical final pH of this system can be calculated.

A polymeric cation exchange material is added to a solution of ceftriaxone to adjust the pH of the solution to a value between that of the second and third pKa's of the drug. An appropriate polymeric ion exchange material with an appropriate pKa is then used to buffer the solution to maintain the pH during storage and/or operation of an electrotransport device

The same approach is used for the drug Cefodizime disodium salt with pKa values of 2.85, 3.37, and 4.18.

## Claims

1. A method for preparing a composition for use in an electrotransport delivery system comprising
providing a drug solution comprising drug ions and associated counterions;
adjusting the pH of the drug solution by contacting the drug solution with a first ion exchange material;
separating the first ion exchange material from the pH-adjusted drug solution;
adding the pH-adjusted drug solution to a reservoir of an electrotransport delivery system; and
contacting the pH-adjusted drug solution with a second ion exchange material.

2. The method of claim 1 wherein the reservoir of the electrotransport delivery system comprises a gel matrix.

3. The method of claim 2 wherein the gel matrix comprises poly(acrylamide), poly(2-hydroxyethyl acrylate), poly(2-hydroxypropyl acrylate), poly(N-vinyl-2-pyrrolidone), poly(n-mmethylol acrylamide), poly(diacetone acrylamide), poly(hydroxylethyl methacrylate), poly(vinyl alcohol), poly(allyl alcohol), polyesters, polycarbonates, polyurethanes, cellulose ethers, methyl cellulose ethers, cellulose and hydroxylated cellulose, methyl cellulose, hydroxylated methyl cellulose, guar, locust, karaya, xanthan, gelatin, or derivatives thereof.

4. The method of claim 3 wherein the gel matrix comprises poly(vinyl alcohol).

5. The method of any of the preceding claims wherein the drug ions are cationic, the associated counterions are anionic, and the first ion exchange material is a first polymeric anion exchange material.

6. The method of any of the preceding claims wherein the drug ions are cationic, the associated counterions are anionic, and the first ion exchange material is a first polymeric anion exchange material and wherein hydroxyl ions are exchanged for the anionic counterions associated with the cationic drug ions when the drug solution is contacted with the first polymeric anion exchange material.

7. The method of any of the preceding claims wherein the pH of the drug solution is adjusted to between pH 3 and pH 9.

8. The method of any of the preceding claims wherein the first polymeric ion exchange material is a first polymeric anion exchange resin.

9. The method of any of the preceding claims wherein the first polymeric ion exchange material is a first polymeric anion exchange resin comprising a hydroxide form of an amine-containing polymer.

10. The method of claim 9 wherein the amine-containing polymer is selected from the group consisting of polyvinyl amines, polyepichlorohydrin/tetraethylenetriamines, copolymers of styrene and divinyl benzene, acrylic/divinyl benzene copolymers, and acrylic/ethyleneglycol dimethacrylate copolymers.

11. The method of any of the preceding claims wherein the first polymeric ion exchange material is a first polymeric anion exchange membrane.

12. The method of any of the preceding claims wherein the second ion exchange material is a second polymeric anion or cation exchange resin.

13. The method of any of the preceding claims wherein the second ion exchange material is a second polymeric ion exchange resin comprising polacrilin, acrylate, methyl sulfonate, methacrylate, carboxylic acid functional groups, sulfonic acid, sulfoisobutyl, or sulfoxyethyl.

14. The method of any of the preceding claims wherein the second ion exchange material comprises polacrilin or acrylate.

15. The method of any of the preceding claims wherein the second ion exchange material is a second polymeric cation exchange resin comprising polyacrylic acid, polyacrylic sulfonic acid, polyacrylic phosphoric acid, or polyacrylic glycolic acid.

16. The method of claim 12 wherein the degree of neutralization of the second polymeric anion or cation exchange resin is 2% to 70%.

17. The method of claim 12 wherein the concentration of the second polymeric anion or cation exchange resin is 20 meq/mL to 200 meq/mL.

18. The method of claim 12 wherein the degree of neutralization of the second polymeric anion or cation exchange resin is 2% to 70% and the concentration of the second polymeric anion or cation exchange resin is 20 meq/mL to 200 meq/mL.

19. The method of any of the preceding claims wherein the drug solution contains cationic drug that is a factor Xa inhibitor and an anti-coagulant.

20. The method of claim 19 wherein the cationic drug is a benzamidine derivative.

21. The method of any of the preceding claims wherein the drug ions are anionic, the associated counterions are cationic, and the first ion exchange material is a first polymeric cationic exchange material.

22. The method of any of the preceding claims wherein the drug solution contains anionic drug ions hydronium ions are exchanged for cationic counterions associated with anionic drug ions when the solution is contacted with the first ion exchange material.

23. The method of any of the preceding claims wherein the first ion exchange material comprises a polymer having one or more acid moieties.

24. The method of any of the preceding claims wherein the drug solution is an aqueous solution.

25. The method of any of the preceding claims wherein the first ion exchange material is separated from the pH-adjusted drug solution by filtration or centrifugation.

## Patentansprüche

1. Verfahren zur Herstellung einer Zusammensetzung zur Verwendung in einem Elektrotransportabgabesystem umfassend
Bereitstellen einer Arzneimittellösung, die Arzneimittelionen und zugehörige Gegenionen aufweist;
Einstellen:des pH-Wertes der Arzneimittellösung durch in Kontakt bringen der Arzneimittellösung mit einem ersten lonenaustauchmaterial;
Trennen des ersten lonenaustauschmaterials von der Arzneimittellösung mit eingestelltem pH-Wert;
Hinzufügen der pH-Wert eingestellten Arzneimittellösung in ein Reservoir eines Elektrotransportabgabesystems; und
in Kontakt bringen der Lösung eingestelltem pH-Wert mit einem zweiten lonenaustauschmaterial.

2. Verfahren nach Anspruch 1, bei dem das Reservoir des Elektrotransportabgabesystems eine Gelmatrix umfaßt.

3. Verfahren nach Anspruch 2, bei dem die Gelmatrix Poly(acrylamid), Poly(2-hydroxylethylacrylat), Poly(2-hydroxypropylacrylat), Poly(N-vinyl-2-pyrrolidon), Poly(n-methylolacrylamid), Poly(diacteonacrylamid), Poly(hydroxylethylmeth-acrylat), Poly(vinylalkohol), Poly(allylalkohol), Polyester, Polycarbonate, Polyharnstoffe, Celluloseether, Methylcelluloseether, Cellulose und hydroxylierte Cellulose, Methylcellulose, hydroxylierte Methylcellulose, Guaran, Johannisbrotgummi, Karayagummi, Xanthan, Gelatine oder deren Derivate umfaßt.

4. Verfahren nach Anspruch 3, bei dem Gelmatrix Poly(vinylalkohol) umfaßt.

5. Verfahren nach einem der vorherigen Ansprüche, bei dem die Arzneimittelionen kationisch sind, die zugehörigen Gegenionen anionisch sind und das erste lonenaustauschmaterial ein erstes polymeres Anionenaustauschmaterial ist.

6. Verfahren nach einem der vorherigen Ansprüche, bei dem die Arzneimittelionen kationisch sind, die zugehörigen Gegenionen anionisch sind und das erste lonenaustauschmaterial ein ersten polymeres Anionenaustauschmaterial ist und bei dem Hydroxylionen durch die anionischen Gegenionen, die mit den kationischen Arzneimittelionen verbunden sind, ausgetauscht werden, wenn die Arzneimittellösung mit dem ersten polymeren Anionenaustauschmaterial in Kontakt gebracht wird.

7. Verfahren nach einem der vorherigen Ansprüche, bei dem der pH-Wert der Arzneimittellösung zwischen pH 3 und pH 9 eingestellt wird.

8. Verfahren nach einem der vorherigen Ansprüche, bei dem das erste polymere lonenaustauchmaterial ein erstes polymeres Anionenaustauschharz ist.

9. Verfahren nach einem der vorherigen Ansprüche, bei dem das erste polymere lonenaustauschmaterial ein erstes polymeres Anionenaustauschharz ist, daß eine Hydroxidform eines aminhaltigen Polymers aufweist.

10. Verfahren nach Anspruch, 9 bei dem das aminhaltige Polymer ausgewählt wird aus der Gruppe, bestehend aus Polyvinylaminen, Polyepichlorhydrin/Tetraethylentriaminen, Copolymeren aus Styrol und Divinylbenzol, Acryl/Divinylbenzolcopolymeren und Acryl/Ethylenglykoldimethacrylatcopolymeren.

11. Verfahren nach einem der vorherigen Ansprüche, bei dem das erste polymere lonenaustauschmaterial eine erste polymere Anionenaustauschmembran ist.

12. Verfahren nach einem der vorherigen Ansprüche, bei dem das zweite lonenaustauschmaterial ein zweites polymeres Anionen- oder Kationenaustauschharz ist

13. Verfahren nach einem der vorherigen Ansprüche, bei dem das zweite Ionenaustauschmaterial ein zweites polymeres Ionenaustauschharz ist, umfassend Polacrilin, Acrylat, Methylsulfonat, Methylacrylat, funktionelle CarbonsäureGruppen, Sulfonsäure, Sulfoisobutyl oder Sulfoxyethyl.

14. Verfahren nach einem der vorherigen Ansprüche, bei dem das zweite lonenaustauschharzmaterial Polacrilin oder Acrylat aufweist.

15. Verfahren nach einem der vorherigen Ansprüche, bei dem das zweite lonenaustauschmaterial ein zweites polymeres Kationenaustauschharz ist, umfassend Polyacrylsäure, Polyacrylsulfonsäure, Polyacrylphosphorsäure oder Polyacrylglykolsäure.

16. Verfahren nach Anspruch 12, bei dem der Neutralisationsgrad des zweiten polymeren Anionen- oder Kationenaustauschharzes 2% bis 70% beträgt.

17. Verfahren nach Anspruch 12, bei dem die Konzentration des zweiten polymeren Anionen- oder Kationenaustauschharzes 20 meq/ml bis 200 meq/ml beträgt.

18. Verfahren nach Anspruch 12, bei dem der Neutralisationsgrad des zweiten polymeren Anionen- oder Kationenaustauschharzes 2% bis 70% beträgt und die Konzentration des zweiten polymeren Anionen- oder Kationenaustauschharzes 20 meq/ml bis 200 meq/ml beträgt.

19. Verfahren nach einem der vorherigen Ansprüche, bei dem die Arzneimittellösung kationische Arzneimittel enthält, das ein Faktor Xa-Inhibitor und eine Anticoagulans ist.

20. Verfahren nach Anspruch 19, bei dem das kationische Arzneimittel ein Benzamidinderivat ist.

21. Verfahren nach einem der vorherigen Ansprüche, bei dem die Arzneimittelionen anionisch:sind, die zugehörigen Gegenionen kationisch sind und das erste lonenaustauschmaterial ein erstes polymeres Kationenaustauschmaterial ist.

22. Verfahren nach einem der vorherigen Ansprüche, bei dem die Arzneimittellösung anionische Ameimittelionen enthält, Hydroniumionen werden durch kationische Gegenionen, die mit den anionischen Arzneimittelmittelionen verbunden sind, ausgetauscht, wenn die Arzneimittellösung mit dem ersten Anionenaustauschmaterial in Kontakt gebracht wird.

23. Verfahren nach einem der vorherigen Ansprüche, bei dem das erste lonenaustauschmaterial ein Polymer umfaßt, das eine oder mehrere Säureanteile hat.

24. Verfahren nach einem der vorherigen Ansprüche, bei dem die Arzneimittellösung eine wässerige Lösung ist.

25. Verfahren nach einem der vorherigen Ansprüche, bei dem das erste lonenaustauschmaterial von der Lösung mit eingestelltem pH-Wert durch Filtration oder Zentrifugation entfernt wird.

## Revendications

1. - Procédé de préparation d'une composition destinée à être utilisée dans un système d'administration par électrotransport comprenant les opérations consistant à :
- se procurer une solution de médicament comprenant des ions de médicament et des contre-ions associés ;
- ajuster le pH de la solution de médicament par mise en contact de la solution de médicament avec une première matière échangeuse d'ions ;
- séparer la première matière échangeuse d'ions de la solution de médicament à pH ajusté ;
- ajouter la solution de médicament à pH ajusté à un réservoir d'un système d'administration par électrotransport ; et
- mettre en contact la solution de médicament à pH ajusté avec une deuxième matière échangeuse d'ions.

2. - Procédé selon la revendication 1, dans lequel le réservoir du système d'administration par électrotransport comprend une matrice de gel.

3. - Procédé selon la revendication 2, dans lequel la matrice de gel comprend le poly(acrylamide), le poly(acrylate de 2-hydroxyéthyle), le poly(acrylate de 2-hydroxypropyle), la poly (N-vinyl-2-pyrrolidone), le poly(N-méthylol acrylamide), le poly(diacétone acrylamide), le poly(méthacrylate d'hydroxyéthyle), le poly(alcool vinylique), le poly(alcool allylique), les polyesters, les polycarbonates, les polyuréthanes, les éthers de cellulose, les éthers de méthyl cellulose, la cellulose et la cellulose hydroxylée, la méthyl cellulose, la méthyl cellulose hydroxylée, la gomme de guar, la gomme de caroube, la gomme de karaya, le xanthane, la gélatine ou des dérivés de ceux-ci.

4. - Procédé selon la revendication 3, dans lequel la matrice de gel comprend le poly(alcool vinylique).

5. - Procédé selon l'une quelconque des revendications précédentes, dans lequel les ions de médicament sont cationiques, les contre-ions associés sont anioniques et la première matière échangeuse d'ions est une première matière échangeuse d'anions polymère.

6. - Procédé selon l'une quelconque des revendications précédentes, dans lequel les ions de médicament sont cationiques, les contre-ions associés sont anioniques et la première matière échangeuse d'ions est une première matière échangeuse d'anions polymère et dans lequel des ions hydroxyle sont échangés contre les contre-ions anioniques associés aux ions de médicament cationiques lorsque la solution de médicament est mise en contact avec la première matière échangeuse d'anions polymère.

7. - Procédé selon l'une quelconque des revendications précédentes, dans lequel le pH de la solution de médicament est ajusté pour être entre pH 3 et pH 9.

8. - Procédé selon l'une quelconque des revendications précédentes, dans lequel la première matière échangeuse d'ions polymère est une première résine échangeuse d'anions polymère.

9. - Procédé selon l'une quelconque des revendications précédentes, dans lequel la première matière échangeuse d'ions polymère est une première résine échangeuse d'anions polymère comprenant une forme hydroxyde d'un polymère à teneur en amine.

10. - Procédé selon la revendication 9, dans lequel le polymère à teneur en amine est choisi dans le groupe constitué par les polyvinyl amines, les polyépichlorohydrine/tétraéthylènetriamines, les copolymères du styrène et du divinyl benzène, les copolymères acrylique/divinyl benzène et les copolymères acrylique/diméthacrylate d'éthylène glycol.

11. - Procédé selon l'une quelconque des revendications précédentes, dans lequel la première matière échangeuse d'ions polymère est une première membrane échangeuse d'anions polymère.

12. - Procédé selon l'une quelconque des revendications précédentes, dans lequel la seconde matière échangeuse d'ions est une seconde résine échangeuse d'anions ou de cations polymère.

13. - Procédé selon l'une quelconque des revendications précédentes, dans lequel la seconde matière échangeuse d'ions est une seconde résine échangeuse d'ions polymère comprenant polacriline, acrylate, méthyl sulfonate, méthacrylate, des groupes fonctionnels acide carboxylique, acide sulfonique, sulfoisobutyle ou sulfoxyéthyle.

14. - Procédé selon l'une quelconque des revendications précédentes, dans lequel la seconde matière échangeuse d'ions comprend polacriline ou acrylate.

15. - Procédé selon l'une quelconque des revendications précédentes, dans lequel la seconde matière échangeuse d'ions est une seconde résine échangeuse de cations polymère comprenant l'acide polyacrylique, l'acide polyacrylique sulfonique, l'acide polyacrylique phosphorique ou l'acide polyacrylique glycolique.

16. - Procédé selon la revendication 12, dans lequel le degré de neutralisation de la seconde résine échangeuse d'anions ou de cations polymère est de 2% à 70%.

17. - Procédé selon la revendication 12, dans lequel la concentration de la seconde résine échangeuse d'anions ou de cations polymère est de 20 méq/ml à 200 méq/ml.

18. - Procédé selon la revendication 12, dans lequel le degré de neutralisation de la seconde résine échangeuse d'anions ou de cations polymère est de 2% à 70% et la concentration de la seconde résine échangeuse d'anions ou de cations polymère est de 20 méq/ml à 200 méq/ml.

19. - Procédé selon l'une quelconque des revendications précédentes, dans lequel la solution de médicament contient un médicament cationique qui est un inhibiteur du facteur Xa et un anti-coagulant.

20. - Procédé selon la revendication 19, dans lequel le médicament cationique est un dérivé de benzamidine.

21. - Procédé selon l'une quelconque des revendications précédentes, dans lequel les ions de médicament sont anioniques, les contre-ions associés sont cationiques et la première matière échangeuse d'ions est une première matière échangeuse de cations polymère.

22. - Procédé selon l'une quelconque des revendications précédentes, dans lequel la solution de médicament contient des ions de médicament anioniques, des ions hydronium sont échangés contre des contre-ions cationiques associés à des ions de médicament anioniques lorsque la solution est mise en contact avec la première matière échangeuse d'ions.

23. - Procédé selon l'une quelconque des revendications précédentes, dans lequel la première matière échangeuse d'ions comprend un polymère ayant une ou plusieurs fractions acides.

24. - Procédé selon l'une quelconque des revendications précédentes, dans lequel la solution de médicament est une solution aqueuse.

25. - Procédé selon l'une quelconque des revendications précédentes, dans lequel la première matière échangeuse d'ions est séparée de la solution de médicament à pH ajusté par filtration ou centrifugation.
